# FASCICULE DE BREVET EUROPEEN

(11) **EP 0 563 372 B1**
(45) Date de publication et mention de la délivrance du brevet: **22.11.1995**
(21) Numéro de dépôt: 92923838.4
(22) Date de dépôt: 15.10.1992
(51) Int. Cl.: A61K 6/06

(54) **PROTHESE DENTAIRE ENTIEREMENT CERAMIQUE A BASE DE SPINELLE ALUMINE/MAGNESIE ET SON PROCEDE DE FABRICATION**
VOLLKERAMISCHE DENTALPROTHESE AUS ALUMINIUMOXYD/MAGNESIUMOXYD SPINELL ENTHALTEND KERAMIK UND VERFAHREN ZU SEINER HERSTELLUNG
DENTAL PROSTHESIS MADE ENTIRELY FROM ALUMINA/MAGNESIA SPINEL BASED CERAMIC, AND METHOD OF MANUFACTURE

(30) Priorité: 15.10.1991 FR 9112673
(43) Date de publication de la demande: 06.10.1993
(73) Titulaire: Tyszblat, Michèle, F-92400 Courbevoie (FR)
(72) Inventeur: Tyszblat, Michèle, F-92400 Courbevoie (FR)
(74) Mandataire: Nony, Michel
(86) Numéro de dépôt international: FR9200974
(87) Numéro de publication internationale: WO9307846

(56) Documents cités:
- EP-A- 0 030 851
- EP-A- 0 241 384
- WO-A-88/08828
- WO-A-89/12436

## Description

La présente invention a pour objet une prothèse dentaire translucide entièrement céramique à base de spinelle alumine/magnésie, ainsi que son procédé de fabrication.

On connait déjà par le brevet européen n° 0 241 384 de la demanderesse un procédé de fabrication de prothèses dentaires, constituées par une infrastructure poreuse obtenue par frittage de particules d'oxydes métalliques, les pores de cette infrastructure étant occupés par un verre infiltré à l'état liquide.

On connait également par la demande de brevet européen 0 030 851 des prothèses céramiques sans retrait contenant des spinelles d'oxyde d'aluminium et d'oxyde de magnésium, mais ces prothèses contiennent également une importante teneur d'oxyde de silicium, de sorte qu'elles ne présentent aucune translucidité, ce qui leur donne un aspect très différent de celui des dents naturelles.

La présente invention a pour objet des prothèses dentaires translucides obtenues par infiltration d'un verre dans les pores d'une infrastructure solide et dont l'infrastructure réalisée à base de spinelle alumine/magnésie peut être conformée soit directement en bouche par le dentiste, soit sur un modèle de la dent par le prothésiste.

Les prothèses selon l'invention présentent l'avantage d'être entièrement céramiques, d'être translucides comme l'émail des dents naturelles, d'être faiblement colorées ou non colorées si on le désire, et d'avoir une résistance mécanique élevée tout en étant parfaitement biocompatibles.

Les prothèses dentaires selon l'invention peuvent avantageusement mais non limitativement être utilisées pour réaliser des pièces connues sous les noms d'"inlay" et d'"onlay" ou encore des couronnes.

La présente invention a pour objet un procédé de fabrication de prothèses dentaires céramiques, translucides comme l'émail des dents, possédant une résistance mécanique élevée et qui sont biocompatibles, ce procédé étant caractérisé par le fait : que l'on réalise une pâte plastique constituée par un mélange de résine, ou de liant, organique avec de fines particules de spinelle alumine/magnésie, d'alumine et de magnésie ; que l'on donne à ladite pâte la forme que doit avoir la prothèse ; que l'on provoque la polymérisation de la résine ou la prise du liant pour fixer la forme de la pâte ; que l'on soumet la pâte ainsi mise en forme à un traitement thermique pour provoquer dans un premier temps la calcination de la résine ou du liant et dans un second temps le frittage réactif des particules de spinelle alumine/magnésie, d'alumine et de magnesie ; que l'on procède éventuellement à la rectification de la forme de l'infrastructure poreuse ainsi obtenue ; et que l'on procède à l'infiltration de cette infrastructure à l'aide d'un verre à l'état fondu.

On sait que les spinelles sont des structures cubiques complexes d'oxydes métalliques.

La spinelle utilisée selon l'invention est la spinelle constituée à partir de quantités voisines de la composition stoechiométrique d'oxyde d'aluminium ou alumine (Al₂O₃) et d'oxyde de magnésium ou magnésie (MgO).

Dans la description qui va suivre cette spinelle sera désignée par l'expression "spinelle MgAl₂O₄" ou spinelle alumine/magnésie.

On sait que cette spinelle se forme lorsque l'on met en présence à haute température un mélange de poudre d'alumine et de magnesie.

Le frittage réactif au cours duquel l'alumine et la magnésie qui, selon l'invention, sont ajoutées à la spinelle MgAl₂O₄ se combine au moins partiellement pour réaliser une quantité supplémentaire de spinelle MgAl₂O₄. Cette réaction entraîne une expansion de volume de l'infrastructure poreuse qui correspond selon le traitement thermique de frittage et la quantité de spinelle supplémentaire formée qui peut être comprise entre 0,6 et 4% de variation de dimension linéaire.

Cette expansion permet de compenser exactement la rétraction de la pâte qui se produit lors de la polymérisation de la résine ou de la prise du liant, rétraction qui peut correspondre par exemple à une réduction de dimension linéaire de 0,1 à 2%, ainsi que la rétraction due à la calcination de la résine ou du liant qui peut correspondre par exemple à une réduction de dimension linéaire de 0,5 à 2%.

En d'autres termes, conformément à l'invention, la charge minérale qui est introduite dans la pâte à laquelle on confère la forme de la prothèse comprend, en-dehors de la spinelle, des additions d'alumine et de magnésie qui, en se combinant lors du frittage réactif, s'expansent pour compenser la rétraction résultant de la polymérisation de la résine ou de la prise du liant puis de leur calcination.

Conformément à l'invention, la résine polymérisable utilisable dans la pâte est une résine organique qui est détruite lors de sa calcination, telles que les résines de type polyépoxyde, polyester ou vinyl ester.

Le liant organique qui est aussi utilisable dans la pâte peut être un ester de cellulose tel que la méthyl cellulose, la méthylhydroxyéthyl cellulose, la méthylhydroxypropyl cellulose, la carboxyméthyl cellulose, ou l'hydroxyéthyl cellulose ou encore l'alginate de sodium ou d'ammonium, tous produits qui sont solubles dans l'eau.

Le liant organique peut être également un éther de cellulose tel que l'éthyl cellulose ou l'acétate cellulose ou encore du polyvinylbutyral qui sont solubles dans des solvants organiques.

Conformément à l'invention, la résine ou le liant doivent autant que possible ne contenir aucun composé tel que de la silice qui ne disparait pas durant la calcination. En effet, de tels composés ont pour effet de faire perdre à la prothèse son caractère translucide qui caractérise les prothèses selon l'invention.

Conformément à un mode de réalisation préféré de l'invention, la charge minérale de la pâte est constituée par un mélange de fines particules comportant en poids de 50 à 84% de spinelle MgAl₂O₄, de 10 à 32% d'alumine et de 6 à 18% de magnésie.

Ces particules ont de préférence une granulométrie inférieure à 40 microns et avantageusement comprise entre 0,5 et 20 microns.

La résine polymérisable telle que définie ci-dessus, à laquelle on peut incorporer la charge minérale, comprend par exemple en poids 40 à 90% de résine poly-époxyde, polyester ou vinyl ester, 0 à 80% de diluant réactif monomère, 0,5 à 5% de catalyseur, 0 à 4% d'accélérateur et 0 à 10% de plastifiant.

Le liant organique tel que défini ci-dessus, auquel on peut incorporer la charge minérale peut comprendre par exemple de 3 à 15% en poids de liant organique, jusqu'à 10% en poids de plastifiant et de 75 à 95% en poids d'eau ou de solvant organique.

La pâte à laquelle on donne la forme de la prothèse contient avantageusement de 55 à 78% en volume d'oxydes métalliques, le reste étant constitué par la résine ou le liant défini ci-dessus.

Conformément à l'invention, la pâte ainsi obtenue peut être utilisée directement par le dentiste qui l'applique sur la dent préalablement préparée qui doit recevoir la prothèse et lui donne la forme que doit avoir la prothèse. Dans ce cas, le liant effectue sa prise en bouche tandis que la résine subit une photopolymérisation en bouche, puis, après démoulage, une thermopolymérisation. Dans une variante, la résine subit en bouche une chémopolymérisation, c'est-à-dire une polymérisation par voie chimique.

Il est également possible de conférer à la pâte la forme que doit avoir la prothèse en l'appliquant sur un modèle de plâtre puis en procédant à la thermopolymérisation de la résine ou à la prise du liant, puis en démoulant ce qui peut être fait dans le laboratoire d'un prothésiste.

Lorsque l'on souhaite réaliser en bouche une prothèse telle que par exemple une couronne, il peut être intéressant d'utiliser une coquille céramique poreuse qui est facilement usinable. Une telle coquille peut être constituée par 70 à 100% en poids de spinelle MgAl₂O₄, jusqu'à 30% en poids de whiskers tels que de l'alumine, de la magnésie ou de la mullite (3Al₂O₃-2SiO₂) et jusqu'à 20% en poids de fibres d'alumine ou de silice. Après mise en forme par injection, cette composition est soumise à un frittage de 1200 à 1400°C. Elle présente alors une porosité ouverte de 10 à 30% en volume.

Cette coquille constituera la surface externe de la prothèse, ce qui facilite et simplifie grandement la mise en forme de la pâte par le dentiste pour réaliser l'infrastructure.

Lorsque la pâte est mise en forme dons un laboratoire, cette mise en forme s'effectue en prenant les empreintes traditionnelles qui sont utilisées pour les fabrications de prothèses.

Conformément à l'invention, le frittage de l'ébauche ainsi obtenue s'effectue par exemple dans un four à air en provoquant dans un premier temps la calcination de la résine ou du liant par une élévation de la température à 4/500°C en 1 à 8 heures. Conformément à l'invention, cette calcination s'effectue en laissant un minimum de résidu solide.

Dans un second temps, la température est portée à environ 1050/1200°C en 1 heure, puis elle est maintenue constante pendant par exemple 1 à 4 heures pour provoquer le frittage.

Après refroidissement, il ne subsiste qu'une infrastructure poreuse réalisée principalement en spinelle MgAl₂O₄, l'alumine et la magnésie qui existaient dans le mélange de poudre initial ayant partiellement réagi l'une sur l'autre pour donner un supplément de spinelle.

On procède à la rectification de l'infrastructure si il est nécessaire de modifier et d'adapter sa forme.

Enfin, on réalise l'infiltration de l'infrastructure poreuse par un verre porté à l'état liquide à haute température. Cette infiltration s'effectue par exemple en plaçant l'infrastructure poreuse dans une coupelle sur la poudre de verre en élevant à l'air la température à 6/700°C en 3 à 10 minutes puis sous vide en portant la température à 1100/1200°C en 10 à 60 minutes, après quoi la température est maintenue sous vide à un palier pendant par exemple 20 minutes à 2 heures.

Le verre peut être constitué avantageusement par un mélange de 12 à 30% de silice (SiO₂), de 3 à 20% d'alumine (Al₂O₃), de 3 à 20% d' oxyde de bore (B₂O₃), de 15 à 45% d' oxyde de lanthane (La₂O₃), de 0 à 15%, d'oxyde d'yttrium (Y₂O₃), de 0 à 8% d'oxyde de titane (TiO₂) et de 0 à 4% d'oxydes colorants.

Lorsque cela est nécessaire, on retire l'excès de verre par sablage.

Enfin, l'on procède à un émaillage en volume avec une céramique dont le coefficient de dilatation est adapté à celui de la prothèse ainsi obtenue. Dans une variante, on se borne à réaliser un polissage de la prothèse qui est elle-même translucide.

La présente invention a également pour objet une prothèse dentaire céramique, biocompatible, présentant une translucidité analogue à celle des dents ainsi qu'une bonne résistance mécanique, caractérisée par le fait qu'elle a été obtenue par le procédé décrit ci-dessus et qu'elle est constituée par une infrastructure poreuse rigide obtenue par frittage réactif de fines particules de spinelle alumine/magnésie, d'alumine et de magnésie, les pores de ladite structure étant occupés par un verre infiltré ultérieurement à température élevée.

La composition globale en poids de la prothèse céramique selon l'invention peut être par exemple la suivante :

| | |
|---|---|
| Spinelle MgAl₂O₄ | 45 à 70% |
| Alumine | 8 à 20% |
| Magnésie | 3 à 12% |
| Verre | 20 à 40% |

La prothèse selon l'invention peut avantageusement être émaillée en volume avec une céramique dont le coefficient de dilatation est adapté à celui de la prothèse.

Dans le but de mieux faire comprendre l'invention, on va en décrire maintenant à titre d'illustration et sans aucun caractère limitatif, plusieurs exemples de réalisation décrits ci-après.

### Exemple 1

Pour réaliser un inlay en bouche destiné à combler une perte de substance qui n'intéresse pas une cuspide, on procède de la manière suivante.

On prépare tout d'abord la cavité dentaire en la taillant par fraisage et en lui donnant une dépouille convenable.

On réalise ensuite une pâte dont la composition en poids est la suivante :

### Charge d'oxydes métalliques

| | |
|---|---|
| Spinelle MgAl₂O₄ | 70% |
| Alumine | 19% |
| Magnésie | 11% |

### Résine

| | |
|---|---|
| - Vinyl ester | 66,5% |
| - Diluant réactif monomère(triéthylèneglycol -diméthacrylate) | 30% |
| - Catalyseur (benzoyl peroxyde) | 2% |
| - Activateur de photopolymérisation (1-2 diketone benzyl camphro quinone) | 1,5% |

On réalise la pâte en mélangeant 65 volumes de charge dans 35 volumes de résine.

On place en bouche la pâte ainsi obtenue de manière à obturer la cavité réalisée dans la dent et on donne à la partie supérieure de la pâte la forme que doit avoir la prothèse.

On procède à la photopolymérisation a l'aide d'une lampe à rayonnement ultraviolet.

On démoule l'ébauche de prothèse ainsi obtenue, puis on procède à sa thermopolymérisation en la chauffant pendant 1 heure à 80°C, puis pendant 1 heure à 110°C.

On procède ensuite au frittage en portant l'ébauche à 100°C en 3 heures, ce qui détruit la résine, puis à 1120°C en 1 heure, température que l'on maintient pendant 2 heures.

On procède ensuite à l'infiltration du verre qui a la composition en poids suivante :

| | |
|---|---|
| -SiO₂ | 20% |
| -Al₂O₃ | 12% |
| -B₂O₃ | 3% |
| -La₂O₃ | 40% |
| -TiO₂ | 7% |
| Oxydes colorants | 2% |

On place dans une coupelle l'ébauche de prothèse sur la poudre de verre.

On porte la température du four à 600°C en 6 minutes, on réalise le vide dans le four et l'on porte sa température à 1140°C en 15 minutes, l'on maintient le four à 1140°C pendant 30 minutes et l'on refroidit en 10 minutes.

On procède ensuite au sablage de la prothèse pour éliminer les excès de verre puis à son polissage.

Elle est ensuite scellée ou collée en bouche selon la technique habituelle.

### Exemple 2

Pour réaliser une couronne selon l'invention directement mise en forme en bouche par le dentiste, on opère de la manière, suivante.

Le dentiste prépare la dent en la taillant et en procedant au fraisage de son périmètre pour réduire sa hauteur et réaliser une dépouille convenable.

Il choisit ensuite une coquille dentaire préformée qui correspond à la forme externe que doit avoir la couronne. De telles coquilles sont disponibles dans le commerce. Elles contiennent par exemple 80% en poids de spinelle MgAl₂O₄ et 20% en poids de whiskers(mullite 3Al₂O₃-2SiO₂).

Le dentiste met en place la coquille en adaptant sa forme au cas clinique, soit par fraisage ou par apport de matière à l'aide d'un composite polymérisable.

Il prépare une pâte ayant la composition pondérale suivante :

### Charge d'oxyde métalliques

| | |
|---|---|
| Spinelle MgAl₂O₄ | 75% |
| Alumine | 16% |
| Magnésie | 9% |

### Résine

### A.Composé de base

| | |
|---|---|
| -Vinyl ester | 57% |
| -Diluant réactif (monomère diuréthane-diméthacrylate) | 40% |
| -Accélérateur | 3% |

### B.Catalyseur

| | |
|---|---|
| -Vinyl ester | 57% |
| -Diluant réactif (monomère diuréthane-diméthacrylate) | 40% |
| -Catalyseur (péroxyde de benzoyle) | 3% |

On réalise la pâte en mélangeant avant l'emploi 62, volumes de charge, 38 volumes de résine (constituants A et B à égalité).

Le dentiste remplit la coquille de cette pâte puis il l'a met en place sur la dent.

La chémopolymérisation achevée, il procède au démoulage.

Après la finition des limites, le prothésiste procède à un traitement thermique qui consiste à porter la température de l'ébauche de prothèse à 400°C en 6 heures pour détruire la résine, puis à 1160°C en 1 heure 30. Cette température est maintenue pendant 2 heures, après quoi on laisse refroidir l'ébauche.

Il procède ensuite à l'infiltration de l'infrastructure à l'aide d'un verre ayant la composition pondérale suivante :

| | |
|---|---|
| -SiO₂ | 20% |
| -Al₂O₃ | 20% |
| -B₂O₃ | 12% |
| -La₂O₃ | 40% |
| -TiO₂ | 8% |

L'imprégnation s'effectue comme indiqué précédemment en portant la température à 700°C en 10 minutes à l'air, puis en faisant le vide et en portant la température à 1150°C en 30 minutes et en maintenant cette température pendant 1 heure, après quoi, on laisse refroidir la prothèse qui est alors polie.

### Exemple 3

Pour réaliser selon l'invention un onlay destiné au comblement d'une perte de substance partielle intéressant une cuspide on procède de la manière suivante :

Le dentiste prend une empreinte de la dent, puis reconstitue sa morphologie avec de la cire ou de la résine et réalise une gouttière transparente par thermoformage, il prépare ensuite la dent par fraisage et lui donne la dépouille convenable.

Il prépare une pâte chémopolymérisable ayant la composition suivante :

### Charge d'oxydes métalliques

| | |
|---|---|
| -Spinelle MgAl₂O₄ | 84% |
| -Alumine | 10% |
| -Magnésie | 6% |

### Résine

### A. Pâte de base

| | |
|---|---|
| -Vinyl ester | 63% |
| -Diallyl phthalate | 32% |
| -Diméthyl-para-toluidine | 3% |

### B.Catalyseur

| | |
|---|---|
| -Vinyl ester | 65% |
| -Diallyl phthalate | 32% |
| -Péroxyde de benzoyle | 3% |

La pâte est réalisée en mélangeant avant l'emploi 72 volumes de charge avec 14 volumes de la pâte de base A et 14 volumes de catalyseur B.

Le dentiste procède alors à la mise en place sur la dent de la gouttière remplie de la pâte. Lorsque la chémopolymérisation est terminée, il procède au démoulage de l'ébauche de prothèse. Le prothésiste procède ensuite au traitement thermique de l'ébauche en la portant à 400°C en 5 heures pour détruire la résine, puis à 1190°C en 2 heures, température à laquelle il la maintient pendant 2 heures.

Il procède ensuite à son refroidissement naturel dans le four.

L'infrastructure est ensuite imprégnée de verre en procedant comme indiqué dans les exemples précédents, le verre utilisé a la composition en poids suivante :

| | |
|---|---|
| -SiO₂ | 15% |
| -B₂O₃ | 8% |
| -Al₂O₃ | 20% |
| -La₂O₃ | 30% |
| -Y₂O₃ | 15% |
| -TiO₂ | 8% |
| -Oxydes colorants | 4% |

L'infiltration est réalisée en portant l'ébauche à 700°C en 6 minutes sous air, puis en élevant la température à 1180°C en 20 minutes sous vide. On maintient la température de 1180°C pendant 15 minutes sous vide et on refroidit sous air pour ramener à la température ambiante en 10 minutes.

On procède ensuite au retrait des excès de verre et à la finition par polissage et la prothèse est ensuite fixée par collage ou scellement.

### Exemple 4

Pour réaliser en laboratoire un inlay sur un modèle de dent préalablement préparée, on opère de la manière suivante :

Le dentiste réalise la prise de l'empreinte de la dent préparée qui est adressée au laboratoire.

Le prothésiste doit utiliser un plâtre non rétractable (moins de 0,1%) qui peut être porté pendant 2 heures à la température de 100 à 120°C.

Il réalise ensuite le modèle en plâtre de la dent préparée, puis procède au comblement des contre-dépouilles avec de la cire.

Il applique un vernis d'espacement selon la technique traditionnelle, puis un agent de démoulage constitué par exemple par de l'huile de silicone non réactive.

Le prothésiste procède ensuite au comblement de la cavité d'inlay sur le modèle en plâtre à l'aide d'une pâte qu'il prépare et qui a la composition pondérale suivante :

### Charge d'oxydes métalliques

| | |
|---|---|
| - Spinelle MgAl₂O₄ | 50% |
| - Alumine | 32% |
| - Magnésie | 18% |

### Résine polyester insaturée

| | |
|---|---|
| - Résine orthophtalique | 68% |
| - Monomère copolymérisable (diallyl phthalate) | 30% |
| - Catalyseur (Ethyl-2 perhéxanoate de butyle tertiaire) | 2% |

La pâte est obtenue en mélangeant avant l'emploi 55 volumes de charge à 45 volumes de résine.

Le prothésiste applique la pâte et la sculpte.

Il procède ensuite à sa thermopolymérisation en chauffant l'ébauche de prothèse pendant 1 heure à 90°C.

Après refroidissement il procède au démoulage.

Pour réaliser le frittage réactif de l'infrastructure, on porte sa température à 500°C en 3 heures pour détruire la résine, puis à 1080°C en 1 heure, température que l'on maintient pendant 4 heures avant de laisser refroidir.

On procède ensuite à l'imprégnation avec un verre ayant la composition pondérale suivante :

| | |
|---|---|
| -SiO₂ | 18% |
| -Al₂O₃ | 10% |
| -B₂O₃ | 18% |
| -La₂O₃ | 30% |
| -Y₂O₃ | 15% |
| -TiO₂ | 8% |
| -Oxydes colorants | 1% |

L'infiltration s'effectue en élevant la température à 650°C en 6 minutes à l'air, puis à 1120°C en 10 minutes sous vide. La température est maintenue à 1120°C sous vide pendant 25 minutes, puis la prothèse est refroidie en 5 minutes.

Le prothésiste procède ensuite à la finition de la prothèse en éliminant les excès de verre et en réalisant son polissage.

La prothèse ainsi obtenue est ensuite fixée sur la dent par collage ou scellement.

### Exemple 5

Pour réaliser selon l'invention une couronne entièrement en céramique au laboratoire du prothésiste, on opère de la manière suivante.

Le dentiste prépare la dent en lui donnant sa taille et sa dépouille et prend son empreinte selon la technique conventionnelle.

Le prothésiste réalise un modèle de la dent avec du plâtre. Il applique sur le modèle un vernis d'espacement puis un agent de démoulage constitué par de l'huile de silicone non réactive.

Il choisit ensuite une coquille dentaire préformée correspondant à la configuration de la couronne, et la rectifie pour l'adapter à la denture du patient par soustraction de matière par fraisage ou par addition de matière.

Il remplit ensuite la coquille avec un pâte thermopolymérisable ayant la composition pondérale suivante :

### Charge d'oxydes métalliques

| | |
|---|---|
| -Spinelle MgAl₂O₄ | 80% |
| -Alumine | 13% |
| -Magnésie | 7% |

### Résine époxyde

| | |
|---|---|
| -Diglycidylether du bisphenol A | 81,5% |
| -Diluant réactif (1-4 Butane diol diglycidyl ether) | 15% |
| -Catalyseur (BF3:MEA complexe trifluorure de bore monoéthylamine) | 3,5% |

La pâte est réalisée juste avant l'emploi en mélangeant 66 volumes de charge à 34 volumes de résine.

Après avoir rempli la coquille avec la pâte, le prothésiste la met en place sur le modèle et il procède à l'élimination des excès de pâte, et à la finition des limites.

Il effectue ensuite la thermopolymérisation en chauffant pendant une heure à la température de 130°C, puis après démoulage, il procède à un nouveau chauffage d'une heure à la température de 180°C.

Le prothésiste porte ensuite l'ébauche de prothèse à 500°C en 8 heures pour détruire la résine, puis a 1170°C en 1 heure, temperature qu'il maintient pendant 2 heures, puis il laisse refroidir la prothèse dans le four.

Il essaie ensuite l'infrastructure ainsi obtenue en l'appliquant sur le modèle et il procède aux rectifications qui pourraient être nécessaires.

L'infrastructure est ensuite, selon la méthode décrite ci-dessus, infiltrée d'un verre ayant la composition suivante :

| | |
|---|---|
| -SiO₂ | 20% |
| -Al₂O₃ | 12% |
| -B₂O₃ | 8% |
| -La₂O₃ | 45% |
| -Y₂O₃ | 5% |
| -TiO₂ | 6% |
| -Oxydes colorants | 4% |

L'infiltration est réalisée en élevant sa température à 600°C pendant 10 minutes à l'air, puis en la portant à 1150°C sous vide en 40 minutes après avoir maintenu la température à 1150°C pendant 30 minutes sous vide, en laisse refroidir.

Il suffit ensuite au prothésiste d'éliminer les excès de verre et de pratiquer le polissage.

La couronne peut être ensuite posée par collage ou scellement.

### Exemple 6

On réalise un inlay selon l'invention en opérant comme indiqué à l'exemple 4, si ce n'est que l'on utilise une pâte ayant la composition pondérale suivante :

### Charge

| | |
|---|---|
| -Spinelle MgAl₂O₄ | 84% |
| -Alumine | 10% |
| -Magnésie | 6% |

### Résine

| | |
|---|---|
| -Vinyl ester | 50% |
| -Diluant monomère (Triéthylèneglycol diméthacrylate) | 48% |
| -Catalyseur (peroxyde de benzoyle) | 2% |

La pâte est obtenue en mélangeant 75 volumes de charge à 25 volumes de résine.

La thermopolymérisation est réalisée sur le modèle de dent dans une étuve pendant 1 heure à 80°C, puis pendant 1 heure à 120°C.

Après refroidissement et démoulage, on réalise le frittage en élevant la température à 400°C en 3 heures pour détruire la résine, puis en la portant à 1060°C en 1 heure. On maintient ensuite cette température de 1060°C pendant 4 heures et on laisse refroidir.

Le prothésiste procède ensuite au contrôle et si besoin à la rectification de l'infrastructure.

Il réalise ensuite l'infiltration d'un verre dont la composition pondérale est la suivante :

| | |
|---|---|
| -SiO₂ | 20% |
| -B₂O₃ | 18% |
| -Al₂O₃ | 9% |
| -La₂O₃ | 45% |
| -TiO₂ | 4% |
| -Oxydes colorants | 4% |

Le cycle thermique d'infiltration consiste à élever la température à 700°C en 3 minutes en présence d'air, puis à porter la température à 1100°C en 15 minutes sous vide. La température est maintenue à 1100°C pendant 12 minutes sous vide, puis la prothèse est refroidie en 8 minutes à l'air.

Le prothésiste élimine les excès de verre par sablage.

Il procède ensuite à l'émaillage en volume de la prothèse à l'aide de céramique dentaire classique adaprée au coefficient de dilatation de l'infrastructure en utilisant par exemple une céramique ayant la composition pondérale suivante :

| | |
|---|---|
| -Oxyde de sodium (Na₂O) | 4,7 à 4,2% |
| -Oxyde de potassium (K₂O) | 8,2 à 6,8% |
| -Oxyde de calcium (CaO) | 1,8 à 1,5% |
| -Oxyde d'aluminium (Al₂O₃) | 13 à 15% |
| -Oxyde de silicium (SiO₂) | 62,8 à 68% |
| -Oxyde de bore (B2O₃) | 7,5 à 6,5% |

La prothèse est ensuite fixée sur la dent par scellement ou collage.

### Exemple 7

On réalise une prothèse d'onlay selon l'invention en procédant comme indiqué à l'exemple 4, si ce n'est que l'on utilise une pâte ayant la composition pondérale suivante :

### Charge

| | |
|---|---|
| -Spinelle MgAl₂O₄ | 70% |
| -Alumine | 20% |
| -Magnésie | 10% |

### Résine

| | |
|---|---|
| -Vinylester | 60% |
| -Styrène | 38% |
| -Catalyseur (ter-butyl-perbenzoate) | 1,8% |
| -Accélérateur (dimethyl-aniline) | 0,2% |

La pâte est réalisée avant l'emploi en mélangeant 65 volumes de charge avec 35 volumes de résine.

On réalise la thermopolymérisation de l'infrastructure sur le modèle de dent dans une étuve pendant 1 heure à 100°C, puis pendant 1 heure à 120°C.

Après démoulage, on procède au frittage en portant la température à 460°C en 4 heures pour détruire la résine, puis à 1120°C en 1 heure. On maintient la température de 1120°C pendant 2 heures et on laisse refroidir.

On procède si besoin à la rectification de l'infrastructure, puis, comme indiqué précédemment à son infiltration avec un verre ayant la composition pondérale suivante

| | |
|---|---|
| -SiO₂ | 80% |
| -B₂O₃ | 20% |
| -Al₂O₃ | 6% |
| -La₂O₃ | 25% |
| -Y₂O₃ | 15% |
| -TiO₂ | 4% |

avec un cycle thermique consistant à élever la température à 600°C en 6 minutes en présence d'air et à porter ensuite la température à 1140°C en 10 minutes sous vide. La température de 1140°C est maintenue sous vide pendant 20 minutes, puis elle est suivie d'un refrodissement à l'air en 10 minutes.

On élimine ensuite les excès de verre par sablage et on réalise un émaillage en volume avec de la céramique dentaire présentant un coefficient de dilatation compatible avec celui de la prothèse.

On peut utiliser à cet effet une céramique ayant la composition pondérale suivante :

| | |
|---|---|
| -Oxyde de sodium | 4,5% |
| -Oxyde de potassium | 7,5% |
| -Oxyde de calcium | 1,7% |
| -Oxyde d'aluminium | 14% |
| -Oxyde de silicium | 65,3% |
| -Oxyde de bore | 7% |

On procède enfin à la mise en place de l'onlay par scellement ou collage.

### Exemple 8

Pour réaliser en laboratoire un inlay selon l'invention, on procède comme indiqué à l'exemple 4 en réalisant une pâte à partir de :

### Charge d'oxydes métalliques

| | |
|---|---|
| -Spinelle MgAl₂O₄ | 84% |
| -Alumine | 10% |
| -Magnésie | 6% |

### Liquide

| | |
|---|---|
| -H₂O | 88% |
| -Liant organique (méthyl cellulose) | 7% |
| -Plastifiant (polyéthylène glycol 400) | 5% |

Les compositions ci-dessus sont données en poids.

On prépare la pâte au moment de l'emploi en mélangeant 70 volumes de charge avec 30 volumes de liquide.

On procède au modelage de l'inlay, puis à son séchage pendant 1 heure dans une étuve à 80°C.

Après démoulage, on procède au frittage de l'infrastructure en la portant à 500°C en 1 heure pour détruire le liant, puis à 1130°C en 1 heure, température que l'on maintient pendant 2 heures.

Après refroidissement, on procède ci nécessaire à une rectification de l'infrastructure que l'on traite ensuite comme indiqué à l'exemple 4.

### Exemple 9

Pour réaliser un inlay selon l'invention on procède comme indiqué à l'exemple 4, si ce n'est que l'on constitue la pâte à partir de :

### Charge d'oxydes métalliques

| | |
|---|---|
| -Spinelle Mg^{A}l₂O₄ | 73% |
| -Alumine | 18% |
| -Magnésie | 9% |

### Liquide

| | |
|---|---|
| -H₂O | 85% |
| -Liant (méthylhydroxypropyl cellulose) | 15% |

Les compositions données ci-dessus sont des compositions pondérales.

On réalise la pâte en mélangeant juste avant l'emploi 63 volumes de charge d'oxydes métalliques avec 37 volumes de liquide.

Dans une variante, le liquide peut être constitué par :

| | |
|---|---|
| -Solvant (2/3 trichlore éthylène-1/3 éthanol) | 85% |
| -Liant (éthyl cellulose) | 15% |

L'ébauche constituée à l'aide de cette pâte est séchée pendant 1 heure dans une étuve à 80°C, puis démoulée.

Elle est ensuite soumise à un frittage en étant portée à 500°C en 2 heures pour détruire le liant, puis à 1150°C en 1 heure, température que l'on maintient pendant 2 heures.

Après refroidissement on procède comme indiqué à l'exemple 6.

### Exemple 10

Pour réaliser un onlay selon l'invention, on procède comme indiqué à l'exemple 4 mais en constituant la pâte à partir de :

### Charge d'oxydes métalliques

| | |
|---|---|
| -Spinelle Mg^{A}l₂O₄ | 78% |
| -Alumine | 14% |
| -Magnésie | 8% |

### Liquide

| | |
|---|---|
| -H₂O | 87% |
| -Liant (alginate d'ammonium) | 3% |
| -Plastifiant (polypropylène glycol) | 10% |

Les pourcentages indiqués ci-dessus sont donnés en poids.

On prépare la pâte en mélangeant au moment de l'emploi 65 volumes de charge à 35 volumes de liquide.

Dans une variante le liquide peur être constitué par :

| | |
|---|---|
| -Plastifiant (octyl phtalate) | 10% |
| -Solvant organique (mélange de 63% de trichloréthylène et de 24% d'éthanol) | 87% |
| -liant (polyvinyl butyral) | 3% |

Après modelage de l'inlay on procède à son séchage pendant 30 minutes à 80°C dans une étuve.

Après démoulage, on réalise le frittage en portant l'ébauche à 450°C en 1 heure 30 pour détruire le liant, puis à 1150°C en 1 heure, température que l'on maintient pendant 2 heures.

Après refroidissement, on procède comme indiqué à l'exemple 7.

Dans tous les exemples de réalisation décrits ci-dessus, on obtient des prothèses céramiques biocompatibles, présentant une translucidité similaire à celle des dents et une bonne solidité mécanique.

## Revendications

1. Procédé de fabrication de prothèses dentaires céramiques, translucides comme l'émail des dents, possédant une résistance mécanique élevée et qui sont biocompatibles, ce procédé étant caractérisé par le fait : que l'on réalise une pâte plastique constituée par un mélange de résine, ou de liant, organique avec de fines particules de spinelle alumine/magnésie, d'alumine et de magnésie ; que l'on donne à ladite pâte la forme que doit avoir la prothèse ; que l'on provoque la polymérisation de la résine ou la prise du liant organique pour fixer la forme de la pâte ; que l'on soumet la pâte ainsi mise en forme à un traitement thermique pour provoquer dans un premier temps la calcination de la résine ou du liant et dans un second temps le frittage réactif des particules de spinelle alumine/magnésie, d'alumine et de magnésie ; que l'on procède éventuellement à la rectification de la forme de l'infrastructure poreuse ainsi obtenue ; et que l'on procède à l'infiltration de cette infrastructure à l'aide d'un verre à l'état fondu.

2. Procédé selon la revendication 1, caractérisé par le fait que les quantités d'alumine et de magnésie présentes lors du frittage réactif sont telles que dans les conditions de frittage elles donnent lieu à la formation d'une quantité supplémentaire de spinelle alumine/magnésie en provoquant une expansion de l'infrastructure qui compense sa contraction résultant de la polymérisation ou de la prise ainsi que de la calcination de la résine ou du liant.

3. Procédé selon l'une quelconque des revendications 1 et 2, caractérisé par le fait que la pâte est réalisée avec une résine organique polymérisable telle qu'une résine poly-époxyde, polyester ou vinyl-ester qui est détruite lors de sa calcination.

4. Procédé selon la revendication 3, caractérisé par le fait que le durcissement de la pâte est provoqué par la polymérisation de la résine, notamment par photopolymérisation, chémopolymérisation ou thermopolymérisation.

5. Procédé selon l'une quelconque des revendications 1 et 2, caractérisé par le fait que le liant est un liant organique constitué par un ester de cellulose tel que la méthyl cellulose, la méthylhydroxyéthyl cellulose, la méthylhydroxypropyl cellulose, la carboxyméthyl cellulose, ou l'hydroxyéthyl cellulose, un alginate de sodium ou d'ammonium, un éther de cellulose soluble tel que l'éthyl cellulose et l'acétate cellulose, ou encore par du polyvinyl butyral, qui se détruisent lors de la calcination.

6. Procédé selon l'une quelconque des revendications 1 à 5, caractérisé par le fait que la charge minérale de la pâte est constituée par un mélange de fines particules contenant 50 à 84% en poids de spinelle alumine/magnésie, de 10 à 32% en poids d'alumine, et de 6 à 18% en poids de magnésie.

7. Procédé selon l'une quelconque des revendications 3 et 4, caractérisé par le fait que la résine polymérisable comprend de 40 à 90% en poids de résine poly-époxyde, polyester ou vinyl-ester, de 0 à 80% en poids de diluant réactif monomère, et de 0,5 à 5% en poids de catalyseur, de 0 à 4% en poids d'accélérateur, et de 0 à 10% en poids de plastifiant.

8. Procédé selon l'une quelconque des revendications 1 à 7, caractérisé par le fait que les particules de charge d'oxydes métalliques ont une dimension inférieure à 40 microns et de préférence comprise entre 0,5 micron et 20 microns.

9. Procédé selon l'une quelconque des revendications 1 à 8, caractérisé par le fait que la pâte à laquelle on donne la forme de la prothèse contient de 55 à 78% en volume d'oxyde métallique, le reste étant constitué par la résine ou le liant.

10. Procédé selon l'une quelconque des revendications 1 à 9, caractérisé par le fait que l'on donne à la pâte plastique la forme que doit avoir la prothèse en l'appliquant en bouche sur une dent préalablement préparée.

11. Procédé selon l'une quelconque des revendications 1 à 9, caractérisé par le fait que l'on donne à la pâte plastique la forme que doit avoir la prothèse en l'appliquant sur un modèle de plâtre.

12. Procédé selon l'une quelconque des revendications 1 à 11, caractérisé par le fait que l'on utilise une coquille céramique poreuse usinable pour donner à la pâte plastique la forme extérieure que doit avoir la prothèse.

13. Prothèse dentaire céramique, biocompatible, présentant une translucidité similaire à celle de l'émail des dents naturelles ainsi qu' une résistance mécanique élevée, caractérisée par le fait qu'elle a été obtenue par le procédé selon l'une quelconque des revendications 1 à 12 et qu'elle est constituée par une infrastructure poreuse rigide obtenue par frittage réactif de fines particules de spinelle alumine/magnésie, d'alumine et de magnésie, les pores de ladite structure étant occupés par un verre infiltré ultérieurement à température élevée.

14. Prothèse selon la revendication 13, caractérisée par le fait qu'elle est essentiellement constituée par 45 à 70% en poids de spinelle alumine/magnésie, 8 à 20% en poids d'alumine, 3 à 12% en poids de magnésie et 20 à 40% en poids en verre.

15. Prothèse selon l'une des revendications 13 et 14, caractérisée par le fait qu'elle est émaillée en volume avec une céramique dont le coefficient de dilatation est adapté à celui de la prothèse.

## Claims

1. Process for the manufacture of ceramic dental prostheses which are translucent like tooth enamel, which have a high mechanical strength and which are biocompatible, this process being characterized by the fact: that a plastic paste is produced, consisting of a mixture of organic resin, or binder, with fine particles of alumina/magnesia spinel, of alumina and of magnesia; that the said paste is given the form which the prosthesis must have: that the polymerization of the resin or the setting of the organic binder is induced in order to fix the form of the paste; that the paste thus formed is subjected to a heat treatment in order to induce, in a first step, the calcination of the resin or of the binder and, in a second step, the reactive sintering of the particles of alumina/magnesia spinel, of alumina and of magnesia; that the correction of the form of the porous infrastructure thus obtained is optionally carried out; and that the infiltration of this infrastructure with the aid of a glass in the molten state is carried out.

2. Process according to Claim 1, characterized in that the quantities of alumina and of magnesia which are present during the reactive sintering are such that in the conditions of sintering they give rise to the formation of an additional quantity of alumina/magnesia spinel, inducing an expansion of the infrastructure which compensates its shrinkage resulting from the polymerization or from the setting and from the calcination of the resin or of the binder.

3. Process according to either of Claims 1 and 2, characterized in that the paste is produced with a polymerizable organic resin such as a polyepoxide, polyester or vinyl ester resin, which is destroyed during its calcination.

4. Process according to Claim 3, characterized in that the curing of the paste is induced by the polymerization of the resin, especially by photopolymerization, chemical polymerization or thermal polymerization.

5. Process according to either of Claims 1 and 2, characterized in that the binder is an organic binder consisting of a cellulose ester such as methyl cellulose, methyl hydroxyethyl cellulose, methyl hydroxypropyl cellulose, carboxymethyl cellulose or hydroxyethyl cellulose, a sodium or ammonium alginate, a soluble cellulose ether such as ethyl cellulose and cellulose acetate or else of polyvinylbutyral, which are destroyed during the calcination.

6. Process according to any one of Claims 1 to 5, characterized in that the inorganic filler of the paste consists of a mixture of fine particles containing 50 to 84 % by weight of alumina/magnesia spinel, from 10 to 32 % by weight of alumina and from 6 to 18 % by weight of magnesia.

7. Process according to either of Claims 3 and 4, characterised in chat the polymerizable resin includes from 40 to 90 % by weight of polyepoxide, polyester or vinyl ester resin, from 0 to 80 % by weight of monomer reactive diluent and from 0.5 to 5 % by weight of catalyst, from 0 to 4 % by weight of accelerator and from 0 to 10 % by weight of plasticizer.

8. Process according to any one of Claims 1 to 7, characterized in that the metallic oxide filler particles have a dimension smaller than 40 microns and preferably between 0.5 micron and 20 microns.

9. Process according to any one of Claims 1 to 8, characterized in that the paste to which the form of the prosthesis is given contains from 55 to 78 % by volume of metal oxide, the remainder consisting of the resin or the binder.

10. Process according to any one of Claims 1 to 9, characterized in that the plastic paste is given the form which the prosthesis must have by being applied in the mouth to a previously prepared tooth.

11. Process according to any one of Claims 1 to 9, characterized in that the plastic paste is given the form which the prosthesis must have by being applied to a plaster model.

12. Process according to any one of Claims 1 to 11, characterized in that a machinable porous ceramic shell is employed to give the plastic paste the external form which the prosthesis must have.

13. Biocompatible, ceramic dental prosthesis exhibiting a translucency similar to that of the enamel of natural teeth and a high mechanical strength, characterized in that it has been obtained by the process according to any one of Claims 1 to 12 and that it consists of a rigid porous infrastructure obtained by reactive sintering of fine particles of alumina/magnesia spinel, of alumina and of magnesia, the pores of the said structure being occupied by a glass subsequently infiltrated at high temperature.

14. Prosthesis according to Claim 13, characterized in that it essentially consists of 45 to 70 % by weight of alumina/magnesia spinel, 8 to 20 % by weight of alumina, 3 to 12 % by weight of magnesia and 20 to 40 % by weight as glass.

15. Prosthesis according to either of Claims 13 and 14, characterized in that it is enamelled in bulk with a ceramic whose expansion coefficient is adapted to that of the prosthesis.

## Patentansprüche

1. Verfahren zur Herstellung von keramischen Zahnprothesen mit vergleichbarer durchscheinender Beschaffenheit wie Zahnschmelz, die eine erhöhte mechanische Festigkeit besitzen und bioverträglich sind, wobei das Verfahren dadurch gekennzeichnet ist,
daß man eine Kunststoffpaste herstellt, die aus einer Mischung aus einem Harz oder organischen Bindemittel mit feinen Teilchen aus Aluminiummagnesiumspinell, Aluminiumoxid und Magnesiumoxid bestehen; daß man der Paste die Form der vorgesehenen Prothese gibt; daß man die Polymerisation das Harzes oder das Abbinden des organischen Bindemittels zur Fixierung der Form der Paste hervorruft; daß man die auf diese Weise in Form gebrachte Paste einer thermischen Behandlung unterwirft, um in einer ersten Stufe die Calcinierung den Harzes oder des Bindemittels und in einer zweiten Stufe die reaktive Sinterung der Teilchen aus dem Aluminiummagnesiumspinell, dem Aluminiumoxid und dem Magnesiumoxid durchführt; daß man gegebenenfalls eine Korrektur der Form des auf diese Weise erhaltenen porösen Unterbaus vornimmt; und daß man eine Infiltration dieses Unterbaus mit Hilfe eines Glases in geschmolzenem Zustand vornimmt.

2. Verfahren nach Anspruch 1, dadurch gekennzeichnet, daß die Mengen des während der reaktiven Sinterung vorhandenen Aluminiumoxids und Magnesiumoxids so beschaffen sind, daß die Sinterungsbedingungen zur Bildung einer zusätzlichen Menge an Aluminiummagnesiumspinell führen und dabei eine Expansion des Unterbaus hervorrufen, die die Kontraktion aufgrund der Polymerisation oder das Abbindens sowie aufgrund der Calcinierung des Harzes oder des Bindemittels ausgleicht.

3. Verfahren nach einem der Ansprüche 1 und 2, dadurch gekennzeichnet, daß die Paste mit einem organischen polymerisierbaren Harz, wie einem Polyepoxidharz, einem Polyesterharz oder Vinylesterharz, das während der Calcinierung zerstört wird, gebildet ist.

4. Verfahren nach Anspruch 3, dadurch gekennzeichnet, daß die Härtung der Paste durch die Polymerisation des Harzes, insbesondere durch Photopolymerisation, chemische Polymerisation oder thermische Polymerisation hervorgerufen wird.

5. Verfahren nach einem der Ansprüche 1 und 2, dadurch gekennzeichnet, daß es sich beim Bindemittel um ein organisches Bindemittel handelt, das aus einem Celluloseester, wie Methylcellulose, Methylhydroxyethylcellulose, Methylhydroxypropylcellulose, Carboxymethylcellulose oder Hydroxyethylcellulose, einem Natrium- oder Ammoniumalginat, einem löslichen Celluloseether, wie Ethylcellulose oder Celluloseacetat oder aus Polyvinylbutyral besteht, die sich während der Calcinierung zersetzen.

6. Verfahren nach einem dar Ansprüche 1 bis 5, dadurch gekennzeichnet, daß der anorganische Füllstoff der Paste aus einer Mischung von feinen Teilchen mit einem Gehalt an 50 bis 84 Gew.-% Aluminiummagnesiumspinell, 10 bis 32 Gew.-% Aluminiumoxid und 6 bis 18 Gew.-% Magnesiumoxid besteht.

7. Verfahren nach einem der Ansprüche 3 und 4, dadurch gekennzeichnet, daß das polymerisierbare Harz 40 bis 90 Gew.-% Polyepoxid-, Polyester- oder Vinylesterharz, 0 bis 80 Gew.-% monomeres reaktives Verdünnungsmittel und 0,5 bis 5 Gew.-% Katalysator, 0 bis 4 Gew.-% Beschleuniger und 0 bis 10 Gew.-% Weichmacher enthält.

8. Verfahren nach einem der Ansprüche 1 bis 7, dadurch gekennzeichnet, daß die Metalloxid-Füllstoffteilchen eine Größe von weniger als 40 µm und vorzugsweise von 0,5 µm bis 20 µm aufweisen.

9. Verfahren nach einem der Ansprüche 1 bis 8, dadurch gekennzeichnet, daß die Paste, der man die Form der Prothese verleiht, 55 bis 78 Vol.-% Metalloxid enthält, wobei der Rest aus dem Harz oder dem Bindemittel besteht.

10. Verfahren nach einem der Ansprüche 1 bis 9, dadurch gekennzeichnet, daß man der verformbaren Paste die Form der vorgesehenen Prothese verleiht, indem man die im Mund auf einen vorher präparierten Zahn aufbringt.

11. Verfahren nach einem der Ansprüche 1 bis 9, dadurch gekennzeichnet, daß man der verformbaren Paste die vorgesehene Form der Prothese verleiht, indem man sie auf ein Gipsmodell aufbringt.

12. Verfahren nach einem der Ansprüche 1 bis 11, dadurch gekennzeichnet, daß man eine bearbeitbare, poröse, keramische Kokille verwendet, um der verformbaren Paste die äußere Form der vorgesehenen Prothese zu verleihen.

13. Bioverträgliche, keramische Zahnprothese mit einer natürlichem Zahnschmelz ähnlichen durchscheinenden Beschaffenheit und einer erhöhten mechanischen Festigkeit, dadurch gekennzeichnet, daß sie nach einem Verfahren gemäß einem der Ansprüche 1 bis 12 erhalten worden ist und aus einem starren, porösen Unterbau gebildet ist, die durch reaktives Sintern feiner Teilchen aus Aluminiummagnesiumspinell, Aluminiumoxid und Magnesiumoxid erhalten worden ist, wobei die Poren dieses Unterbaus durch ein später bei erhöhter Temperatur infiltriertes Glas besetzt sind.

14. Prothese nach Anspruch 13, dadurch gekennzeichnet, daß sie im wesentlichen aus 45 bis 70 Gew.-% Aluminiummagnesiumspinell, 8 bis 20 Gew.-% Aluminiumoxid, 3 bis 12 Gew.-% Magnesiumoxid und 20 bis 40 Gew.-% Glas besteht.

15. Prothese nach einem der Ansprüche 13 und 14, dadurch gekennzeichnet, daß sie mit einem keramischen Material emailliert ist, dessen Ausdehnungskoeffizient an den der Prothese angepaßt ist.
